# EUROPEAN PATENT APPLICATION

(11) **EP 2 589 300 A1**
(43) Date of publication of application: **08.05.2013**
(21) Application number: 10854183.0
(22) Date of filing: 11.11.2010
(51) Int. Cl.: A23L 1/30, A23K 1/16

(54) **BIOLOGICALLY ACTIVE ADDITIVE AND PROCESS FOR PRODUCING SAME**

(30) Priority: 29.06.2010 RU 2010126530
(71) Applicant: Limited Liability Company «GamaVetFarm», Moscow 123098 (RU)
(72) Inventor: NAROVLYANSKIJ, Alexandr Naumovich, Moscow 123098 (RU); PRONIN, Aleksandr Vasil'evich, Moscow 123098 (RU); SANIN, Alexandr Vladimirovich, Moscow 123098 (RU); SVIRIDOV, Aleksej Yur'evich, Moscow 123098 (RU); STARIKOVA, Mariya Valentinovna, Mytischi Moskovskaya obl. 123098 (RU); KHOMICH, Alexandr Vladimirovich, Moscow 105043 (RU)
(74) Representative: von Füner, Nicolai
(86) International application number: PCT/RU2010/000662
(87) International publication number: WO 2012/002835

(57) **Abstract**

The invention relates to dietary and prophylactic biologically active food additives (BAAs), in particular, those consisting of plant raw materials, and to processes for producing the same; it can be used as a food additive for humans and domestic animals to reduce blood cholesterol levels, to stimulate protein glycosylation and to prevent infectious and cardiovascular diseases.

The technical result of the use of the claimed BAA consists in providing a means for reducing blood cholesterol levels, stimulating protein glycosylation and preventing infectious and cardiovascular diseases.

The biologically active additive comprises at least one polyprenyl phosphate or polyprenyl pyrophosphate with from 7 to 30 isoprene units or a mixture of different polyprenyl phosphates and polyprenyl pyrophosphates adsorbed on a solid sorbent, in particular sorbitol, lactose, starch, and also beta-sitosterol.

The biologically active additive is produced by dissolving a mixture of polyprenyl phosphates and/or polyprenyl pyrophosphates in an organic solvent, adding a sorbent to the obtained solution and subsequently mixing and removing the solvent by distillation. Beta-sitosterol can be added along with the sorbent.

## Description

### Field of invention

The invention relates to dietary and prophylactic biologically active food additives (BAAs) from plant raw materials on the basis of polyprenyl phosphates and polyprenyl pyrophosphates and to a process for their production; it may be used as a food additive for humans and domestic animals in order to reduce the blood cholesterol level, to stimulate protein glycosylation and to prevent infectious and cardiovascular diseases.

### Prior art

The applicability of polyprenols and phosphates thereof is limited by the fact that they represent a lipoid mass which is practically insoluble in water and resistant to drying and grinding. Therefore, they are dissolved in a complex solvent and administered only orally or in form of injection. This significantly complicates storage conditions and limits the sphere of application to the field of medicine and veterinary.

In order to expand the application sphere of said medicament, it has to be prepared in form of tablets, capsules or powder suitable for use as an additive to animal forage.

BAAs for the reduction of blood cholesterol levels, stimulation of protein glycosylation and prophylaxis of infectious and cardiovascular diseases are not known to the inventors.

### Disclosure of the invention

The technical result of using the claimed BAA consists in the preparation of a drug for reducing the amount of cholesterol in blood, stimulation of protein glycosylation and prophylaxis of infectious and cardiovascular diseases.

This problem can be solved by applying polyprenols and/or phosphates thereof to biologically inert carriers.

Said technical result is achieved by the fact that a biologically active additive for reducing the cholesterol level in blood, stimulation of protein glycosylation and prophylaxis of infectious and cardiovascular diseases contains at least one polyprenyl phosphate or polyprenyl pyrophosphate having 7 to 30 isoprene units or a mixture of different polyprenyl phosphates and polyprenyl pyrophosphates adsorbed on a solid sorbent, in particular on sorbitol, lactose, and starch.

Said technical result is also achieved by the fact that the content of polyprenyl phosphates in a mixture of polyprenyl phosphates and polyprenyl pyrophosphates amounts to 5-20 wt %.

Said technical result is also achieved by the fact that the content of polyprenyl phosphates in a mixture of polyprenyl phosphates and polyprenyl pyrophosphates amounts to 10 wt %.

Said technical result is also achieved by the fact that the content of the sorbent amounts to 90-99.5 wt %.

Said technical result is also achieved by the fact that the content of a sorbent amounts to 98 wt %.

Said technical result is also achieved by the fact that the biologically active additive further contains beta-sitosterol.

Said technical result is also achieved by the fact that beta-sitosterol content is 1-10 wt%.

Said technical result is also achieved by the fact that beta-sitosterol content is 5 wt %.

Said technical result is also achieved by the fact that the biologically active additive is produced by dissolving a mixture of polyprenyl phosphates and/or polyprenyl pyrophosphates in an organic solvent, adding the sorbent to the obtained solution and subsequent mixing and solvent distillation.

Said technical result is also achieved by the fact that beta-sitosterol is added with the sorbent.

Said technical result is also achieved by the fact that the distillation of the solvent is carried out after mixing.

### Preferred embodiment of the invention

The BAA is obtained as follows:
Polyprenyl phosphates, polyprenyl pyrophosphates or their mixture are dissolved in an organic solvent and mixed with a sorbent. Further, the solvent is distilled out. In the course of distillation polyprenols are adsorbed on the sorbent.

The following process can be presented as an example.

20 g of polyprenyl phosphates are dissolved in 200 g of an organic solvent (acetone, hexane, etc.). 980 g sorbitol, lactose or starch is added to the obtained solution. The mixture is thoroughly mixed, put into a rotary evaporator, and the solvent is distilled out during an hour. The obtained mass is retrieved and placed into a thermostat to get dried at 40°C during 3-4 h. After drying, the solid mass is powdered in a suitable mill and sifted to obtain a fine homogenous powder ready for use.

The BAA is used by adding it to food or forage for animals, or in the form of tablets and capsules.

Some examples demonstrating the effect of the claimed BAA are given below.

### Example 1

The powder prepared according to example 1 (starch is used as a sorbent) is added (450 g/t) to the standard forage for 10-day-old pigs (100 animals). Young pigs of the control group (100 animals) obtain standard forage. After 15 days the safety of the animals from the experimental group amounted to 98.5-100%; the safety of the control group was 97.0-97.8%. Gastroenteric disorders were observed in 0-8 young pigs of the experimental group and in 20-22 animals of the control group. Thus, the application of the BAA essentially reduces the morbidity rate and improves the safety of livestock.

### Example 2

Two groups of 5-10-day-old calves (each consisting of 10 animals) are kept under similar sanitary-hygienic conditions indoors, each group in an individual cage. The animals get pigs will three times a day during a month which is milk in the control group and milk with a powder prepared with lactose (PrenoLakt) as a sorbent (0.125 g per 1 kg of milk) in the experimental group. By the end of the experiment, the mean weight gain is 25.6±1.8 kg per a calf in the test group and 21.2±1.2 kg in the control one; no gastroenteric disorders are observed in animals of the test group; in the control group, they were observed in 5 calves. Thus, the application of the BAA causes weight gain and reduces the morbidity rate in calves.

### Example 3

28-day-old broiler chicken (140000 animals) receive standard food with a powder prepared on the basis of lactose (PrenoLakt) in a dose of 450 g/t of food during 5 days. Chicken in the control group receive standard food. By the end of the experiment, the mortality of animals from the test group significantly decreases (0.07-0.08% versus 0.14% in the control group). Thus, the application of the BAA causes an essential reduction of mortality.

### Example 4. The influence of BAA on the intensity of protein synthesis in macrophages

CBA mice having a mass of 18-20 g receive one oral dose of BAA (10 mg/animal). 1 and 7 days later peritoneal macrophages are obtained and protein production is investigated by using ³H-thymidine labeled amino acids. Table 1 shows that the intensity of protein synthesis significantly increases 7 days after the BAA administration.

**Table 1. The influence of BAA on the intensity of protein synthesis in macrophages**

| Group | Count/min per mg of protein |
|---|---|
| Control | 18947±949 |
| 1 day | 18060±776 |
| 7 days | 53391±7351 |

### Example 5. The influence of BAA on the production of glycoproteins (immunoglobulins)

BAA containing only polyprenyl phosphates (C1), polyprenyl phosphates and polyprenyl pyrophosphates in the ratio 9:1 (C2) or 1:1 (C3) is orally administered to CBA mice having a mass of 18-20 g in a dose of 10 or 50 mg/animal three times: a day before the immunization, simultaneously to the immunization, and a day after the immunization. The animals are immunized intravenously with 4 x 10⁸ of sheep erythrocytes. 5 days later a blood serum is obtained; the titers of antibodies are determined by means of 96-well plates (0.025 ml of physiological solution and 0.025 ml of the serum in different dilutions are poured into each well, 0.025 ml of 1% sheep erythrocytes is added). The plate is incubated during 3 h at 37°C. The maximal serum dilution wherein the agglutination of erythrocytes is still observed is considered to be the antibody titer.

**Table 2. The effects of the BAA on antibody titers towards sheep erythrocytes**

| Group | Antibody titers (log₂±m) | | |
|---|---|---|---|
| | C1 | C2 | C3 |
| Control | 5.3±0.9 | 5.3±0.9 | 5.3±0.9 |
| 10 mg | 5.5±1.2 | 5.8±0.5 | 5.9±0.8 |
| 50 mg | 7.0±0.7 p<0.05 | 7.3±0.8 | 7.2±0.9 |

BAA in a dose of 50 mg significantly increases the antibody titer whose activity is related to the presence of glycosylated immunoglobulins. There is no essential difference between C1, C2 and C3.

### Example 6. The effect of the BAA on the cholesterol level in blood

A group of 5 volunteers aged 42 to 62 years receive 130 mg of BAA on the basis of sorbitol with 5% beta-sitosterol (SitoPren) twice a day during 3 months. The content of cholesterol, high-density lipoproteins (HDL) and low-density lipoproteins (LDL) is analyzed in the beginning of the experiment (0), 22 days later and 3 months after the BAA intake. Whereas the cholesterol content is at the upper limit before the intake of BAA and the LDL content is even somewhat higher than the norm, these parameters have lower values at the same HDL level by the end of the experiment.

**Table 3. The amount of cholesterol and high-density lipoproteins (HDL) and low-density lipoproteins (LDL) in blood of volunteers at different periods after the BAA intake**

| Parameter | Content (mM/ml) | | | |
|---|---|---|---|---|
| | Norm | 0 | 22 days | 3 months |
| Cholesterol | 0-6.0 | 6.0±0.4 | 6.13±0.3 | 5.48±0.21 |
| HDL | 1.03-1.55 | 1.54±0.59 | 1.52±0.17 | 1.54±0.36 |
| LDL | 0-4.1 | 4.66±0.78 | 4.5±0.58 | 4.05±0.12 |

## Claims

1. Biologically active additive for reducing blood cholesterol levels, stimulating protein glycosylation and preventing infectious and cardiovascular diseases containing at least one polyprenyl phosphate or polyprenyl pyrophosphate with a number of isoprene units from 7 to 30, or a mixture of different polyprenyl phosphates and polyprenyl pyrophosphates adsorbed on a solid sorbent, in particular sorbitol, lactose, starch.

2. Biologically active additive according to claim 1, **characterized in that** the content of polyprenyl phosphates in a mixture of polyprenyl phosphates and polyprenyl pyrophosphates amounts to 5-20 wt %.

3. Biologically active additive according to claim 2, **characterized in that** the content of polyprenyl phosphates in a mixture of polyprenyl phosphates and polyprenyl pyrophosphates amounts to 10 wt %.

4. Biologically active additive according to claim 1, **characterized in that** the content of the sorbent is 90.0-99.5 wt %.

5. Biologically active additive according to claim 4, **characterized in that** the content of the sorbent is 98.0 wt %.

6. Biologically active additive according to claim 1, **characterized in that** it further contains beta-sitosterol.

7. Biologically active additive according to claim 6, **characterized in that** the beta-sitosterol content amounts to 1-10 wt %.

8. Biologically active additive according to claim 7, **characterized in that** the beta-sitosterol content is 5 wt %.

9. A process for producing a biologically active additive on the basis of polyprenyl phosphates and/or polyprenyl pyrophosphates, said process consisting in the dissolution of a mixture of polyprenyl phosphates and/or polyprenyl pyrophosphates in an organic solvent, addition of a sorbent to the obtained solution and subsequent mixing and distillation of the solvent.

10. The process for producing a biologically active additive according to claim 9, **characterized in that** beta-sitosterol is added along with the sorbent.

11. The process for producing a biologically active additive according to claim 9 or claim 10, **characterized in that** the distillation of the solvent is carried out after mixing.

## Amended claims

### Amended claims under Art. 19.1 PCT

1. Biologically active additive for reducing blood cholesterol levels and preventing cardiovascular diseases containing at least one polyprenyl phosphates or polyprenyl pyrophosphate with a number of isoprene units from 7 to 30, or a mixture of different polyprenyl phosphates and polyprenyl pyrophosphates adsorbed on a solid sorbent, in particular sorbitol, lactose, starch.

2. Biologically active additive according to claim 1, **characterized in that** the content of polyprenyl phosphates in a mixture of polyprenyl phosphates and polyprenyl pyrophosphates amounts to 5-20 wt %.

3. Biologically active additive according to claim 2, **characterized in that** the content of polyprenyl phosphates in a mixture of polyprenyl phosphates and polyprenyl pyrophosphates amounts to 10 wt %.

4. Biologically active additive according to claim 1, **characterized in that** the content of the sorbent is 90.0-99.5 wt %.

5. Biologically active additive according to claim 4, **characterized in that** the content of the sorbent is 98.0 wt %.

6. Biologically active additive according to claim 1, **characterized in that** it further contains beta-sitosterol.

7. Biologically active additive according to claim 6, **characterized in that** the beta-sitosterol content amounts to 1-10 wt %.

8. Biologically active additive according to claim 7, **characterized in that** the beta-sitosterol content is 5 wt %.

9. A process for producing a biologically active additive on the basis of polyprenyl phosphates and/or polyprenyl pyrophosphates, said process consisting in the dissolution of a mixture of polyprenyl phosphates and/or polyprenyl pyrophosphates in an organic solvent, addition of a sorbent to the obtained solution and subsequent mixing and distillation of the solvent.

10. The process for producing a biologically active additive according to claim 9, **characterized in that** beta-sitosterol is added along with the sorbent.

11. The process for producing a biologically active additive according to claim 9 or claim 10, **characterized in that** the distillation of the solvent is carried out after mixing.
